Europäisches Patentamt

(19) European Patent Office

Office européen des brevets

(11) Veröffentlichungsnummer : **0 359 738 B1**

(12) **EUROPÄISCHE PATENTSCHRIFT**

(45) Veröffentlichungstag der Patentschrift :
02.01.92 Patentblatt 92/01

(51) Int. Cl.⁵ : **A23L 3/34,** A01N 63/02,
A23K 3/00, A24B 15/30

(21) Anmeldenummer : 88901410.6

(22) Anmeldetag : 02.02.88

(86) Internationale Anmeldenummer :
PCT/EP88/00071

(87) Internationale Veröffentlichungsnummer :
WO 88/05992 25.08.88 Gazette 88/19

(54) **ZUBEREITUNG ZUR VERLÄNGERUNG DER HALTBARKEIT VON LEBENSMITTELN, ARZNEIMITTELN UND KOSMETISCHEN PRODUKTEN.**

(30) Priorität : 10.02.87 DE 3704004

(43) Veröffentlichungstag der Anmeldung :
28.03.90 Patentblatt 90/13

(45) Bekanntmachung des Hinweises auf die
Patenterteilung :
02.01.92 Patentblatt 92/01

(84) Benannte Vertragsstaaten :
AT BE CH DE FR GB IT LI NL SE

(56) Entgegenhaltungen :
EP-A- 0 181 562
DE-A- 3 440 735
FR-A- 1 382 194
Patent Abstracts of Japan, vol. 6, no. 118,
(C-111) (996) 2 July 1982 & JP-A-5747466 (A-
sahi Denka Kogyo K.K.) 18 March 1982
Chemical Abstracts, vol. 83, no. 5, 4 August
1975, (Colombus, Ohio, US) see page 385,
abstract 41773m, & JP-A-7542057 (Nishiguchi,
Masaru) 16 April 1975
Chemical Abstracts, vol. 96, no. 21, 24 May
1982, (Colombus, Ohio, US) see page 588,
abstract 179766m, & JP-A-8222681 (Lion Corp.
Asama Chemical Co., LTD) 5 February 1982
Chemical Abstracts, vol. 100, no. 21, 21 May
1984 (Colombus, Ohio, US) see page 519, abstract 173432d, & JP-A-5911168 (Sankyo Enterprise K.K.) 20 January 1984
Chemical Abstracts, vol. 103, no. 8, 26 August
1985 (Colombus, Ohio, US) A.E. El-Nimr: "Lytic
activity of lysozyme relevant to preservativesenzyme interactions", see page 340, abstract
59186p, & Drug Dev. Ind. Pharm. 1985, 11(2-3),
473-80

(56) Entgegenhaltungen :
Chemical Abstracts, vol. 80, no. 15, 15 April
1974, (Colombus, Ohio, US) see page 252,
abstract 81106r, & JP-A-7388225 (Eisai Co.
Ltd.) 19 November 1973
Chemical Abstracts, vol. 84, no. 19, 10 May
1976, (Colombus, Ohio, US) D. Kaplan et al.:
"The effect of lysozyme, singly and in combination with ascorbic acid, on the sensitivity
of Staphylococcus aureus (80) to sodium salicylate, tetracycline and penicillin G.", see
page 93, abstract 130859g, & IRCS Med. Sci.:
Libr. Compend. 1976, 4(2), 90-1

(73) Patentinhaber : HOECHST
AKTIENGESELLSCHAFT
Postfach 80 03 20
W-6230 Frankfurt am Main 80 (DE)

(72) Erfinder : LÜCK, Erich
Robert-Stolz-Strasze 102
W-6232 Bad Soden am Taunus (DE)
Erfinder : KLUG, Christian
Friedrich-Ebert-Strasze 15
W-6231 Schwalbach im Taunus (DE)
Erfinder : LOTZ, Andreas
Coventrystrasze 6
W-6230 Frankfurt am Main 80 (DE)
Erfinder : WÖHNER, Gerhard
Flörsheimer Strasze 27
W-6093 Flörsheim am Main (DE)
Erfinder : VON RYMON LIPINSKI,
Gert-Wolfhard
Hunsrückstrasze 40
W-6230 Frankfurt am Main 80 (DE)

## Beschreibung

Konservierungsstoffe, wie beispielsweise Sorbinsäure, Benzoesäure, Propionsäure, Ameisensäure, p-Hydroxybenzoesäure, Salicylsäure und schweflige Säure werden bekanntlich zur Verlängerung der Haltbarkeit von Lebensmitteln, Arzneimitteln und kosmetischen Produkten eingesetzt. In der Praxis der Lebensmittelkonservierung verstärkt man oft die Wirkung der genannten Konservierungsstoffe durch Zusätze, welche die Wasseraktivität oder den pH-Wert der Lebensmittel erniedrigen, wie Kochsalz, Zucker, Essigsäure oder andere Genußsäuren. Dies kann zu geschmacklichen Veränderungen der Lebensmittel führen, die nicht immer erwünscht sind. Im Falle von Mayonnaisen und Fischmarinaden sowie anderen Feinkostprodukten bevorzugt der Verbraucher mild eingestellte Produkte mit geringem Salz- und Essigzusatz. Allerdings besteht im höheren pH-Bereich die Gefahr, daß sich manche Bakterien verstärkt vermehren und daß sogar für pathogene Keime günstige Lebensbedingungen geschaffen werden. Es treten immer wieder Fälle von Lebensmittelvergiftungen auf, die mit dem Verzehr von nur schwach angesäuerten Produkten in Zusammenhang gebracht werden, da die antimikrobielle Wirkung vieler Konservierungsstoffe mit steigendem pH-Wert deutlich abnimmt.

Enzyme mit N-Acetylmuramidaseaktivität, wie z.B. Lysozym, können ebenfalls zur Konservierung von Lebensmitteln, z.B. Milch-, Fleisch- und Fischprodukten, eingesetzt werden. Mit Hilfe des Verfahrens der Britischen Patentschrift 2 014 032 wird das Wachstum von Mikroorganismen in Milchprodukten durch Konservierung mit Lysozym verhindert. In der Französischen Patentschrift 80 03 321 wird der Einsatz von lysozymartigen Produkten aus Sekreten von Tieren und Pflanzen zur Verhinderung der Spätblähung in verschiedenen Käsesorten dargestellt.

Derartige Produkte können jedoch nicht nur aus Pflanzen und Tieren gewonnen werden, sondern auch aus Streptomyceten wie den Deutschen Offenlegungsschriften 20 11 935, 20 40 444, 21 46 597 und 34 40 735 zu entnehmen ist.

Hidaka et al. (C.A. 80 Zusammenfassung 81106r (1974)) beschreiben eine Zubereitung bestehend aus Zellwand-abbauenden Enzymen in Kombination mit Glycin, Cystin, Cystein, Propylenglycol, Caprylsäuremonoglycerid, Invertase, Vitamin $B_1$-HCl, oder Zitronensäure. Eine derartige Zubereitung kann als Konservierungsmittel Anwendung finden. El-Nimr (C.A. 103 Zusammenfassung 59186p (1985)) beobachteten einen synergistischen Effekt durch Zusammenwirkung von p-Hydroxybenzoesäureester und Lysozym. Kaplan (C.A. 84 Zusammenfassung 130859g) beschreibt zum einen eine Verstärkung der bakteriziden Wirkung von Natriumsalicylat auf Staphylococcus aureus durch Lysozym. Auf der anderen Seite wird aber auch beobachtet, daß Lysozym durchaus desaktivierende Eigenschaften bzw. keine Wirkung auf bakterizide Substanzen, wie z.B. Tetracyclin oder Penicillin hat. Dadurch wird deutlich, daß Lysozym nicht in jeder Vergesellschaftung aktivierende bzw. synergistische Wirkung zeigt.

Im Patent Abstract of Japan 6 118 (1982) wird die konservierende Wirkung durch eine Kombination von proteolytischen Enzymen und Lebensmittelkonservierungsstoffen, gegebenenfalls unter Zugabe von Lysozym, gezeigt. Eine synergistische Wirkung wird jedoch nicht erwähnt.

Es wurde nun überraschend gefunden, daß die Kombination der nicht-enzymatischen Konservierungsstoffe mit Enzymen, die N-Acetylmuramidaseaktivität besitzen, zu einem Wirkungssynergismus führen, der sich darin auszeichnet, daß insbesondere Lebensmittel, Arzneimittel und kosmetische Produkte mit der Mischung vergleichsweise länger konserviert werden können als mit Hilfe der einzelnen Komponenten.

Die Erfindung betrifft somit:

1. Zubereitung enthaltend Benzoesäure und/oder Sorbinsäure oder deren Salze sowie ein oder mehrere Enzyme mit N-Acetylmuramidaseakivität in einem Gewichtsverhältnis von 1 zu 100 bis 10 zu 1.

2. Verwendung der unter 1. charakterisierten Zubereitung zur Anwendung als Konservierungsmittel.

3. Ein Verfahren zur Herstellung der unter 1. charakterisierten Zubereitung, das dadurch gekennzeichnet, daß der nicht-enzymatische Konservierungsstoff und das Enzym mit N-Acetylmuramidaseaktivität in eine geeignete Applikationsform gebracht werden.

Im folgenden wird die Erfindung, insbesondere in ihren bevorzugten Ausführungsformen detailliert beschrieben.

Ferner wird die Erfindung in den Patentansprüchen charakterisiert.

Die zubereitung besteht aus Benzoeäure oder Sorbinsäure oder deren Salzen zusammen mit einem Enzym mit N-Acetylmuramidaseaktivität, das die Fähigkeit besitzt, Bakterienzellen zu lysieren und eine weitere Vermehrung der Mikroorganismen zu verhindern. Bevorzugt geeignete Enzyme sind beispielsweise Lysozym, insbesondere aus Hühnereiweiß, oder bakterienlysierende Enzymprodukte aus Streptomyceten, insbesondere aus Streptomyces coelicolor DSM 3030 sowie dessen Mutanten und Varianten. Das Enzymprodukt aus DSM 3030 gewinnt man leicht nach dem in der Deutschen Offenlegungsschrift 34 40 735 beschriebenen Verfahren. Danach wird Streptomyces coelicolor DSM 3030 in einem Fermentationsmedium unter Zusatz von Zuckerrübenmelasse in einer Menge von 5 bis 50 g, vorzugsweise 10 bis 20 g, pro Liter Kulturmedium kultiviert. Eine weitere Ausbeutesteigerung wird erreicht, wenn man dem Kulturmedium Calciumionen in Form leicht löslicher, ungiftiger Calciumsalze zusetzt, vorzugsweise in Form des preiswerten Calciumchlorids. Vorteilhaft ist eine 0,05 bis 1 molare Calciumionenkonzentration, besonders bevorzugt sind Konzentrationen von 100 bis 500 mmol/l, beispielsweise in Form eines Zusatzes von 0,2 bis 0,5 Gew.-% an Calciumchlorid-Dihydrat.

Die Wirksubstanzen können gleichzeitig oder aufeinanderfolgend Lebensmitteln, wie z.B. Fleisch und Fleischerzeugnissen, Fisch-, Krusten-, Schalen- und Weichtiererzeugnissen, eßbaren gelatineartigen Überzugsmassen für Fleischerzeugnisse, Feinkosterzeugnissen, Flüssigei und flüssigem Eigelb, Gemüse- und Obsterzeugnissen, alkoholischen und alkoholfreien Getränken, Milch und Milcherzeugnissen, Füllungen für Teige und Backwaren, Zucker- und Süßwaren, sowie pharmazeutischen und kosmetischen Produkten, Futtermitteln, Tabak, Tabakerzeugnissen und Verpackungsmaterialien zugegeben werden.

Je nach Anwendungszweck werden ein oder mehrere Enzyme mit N-Acetylmuramidaseaktivität mit Benzoesäure und/oder Sorbinsäure in einem Gewichtsverhältnis von 1 zu 100 bis 100 zu 1, vorzugsweise 1:50 bis 50:1, gemischt und dem zu konservierenden Produkt nach herkömmlichen Verfahren zugesetzt. Die Zugabe der Mischung erfolgt in einer Größenordnung, daß 0,01 bis 2000 U des Enzyms mit N-Acetylmuramidaseaktivität, vorzugsweise 0,1 bis 1000 U, pro mg des zu konservierenden Produkts darin enthalten sind.

Die Kombination von Bensoesäure und/oder Sorbinsäure und Enzymen mit Muramidaseaktivität führt zu einem Wirkungssynergismus. Dies wirkt sich besonders vorteilhaft auf Produkte mit einem milden pH-Wert aus, die beim Verbraucher besonders beliebt sind. Beispielsweise wirkt die Kombination in einem pH-Bereich zwischen 3 bis 6, vorzugsweise 4,5 bis 5,5, besser gegen lebensmittelverderbende und pathogene Mikroorganismen als die Einzelkomponenten für sich allein. Der Vorteil der Mischung besteht darin, daß man den Gehalt der chemischen Konservierungsstoffe in Lebensmitteln, Kosmetika, Pharmazeutika etc. bei gleichzeitiger Verlängerung der Haltbarkeit reduzieren kann. Selbstverständlich kann oder sollte die erfindungsgemäße Wirkstoffkombination nur dann sinnvoll eingesetzt werden, wenn die zu konservierenden Produkte unter hygienisch einwandfreien Bedingungen hergestellt worden sind und eine niedrige Anfangskeimzahl aufweisen.

In den folgenden Beispielen wird die Erfindung weitergehend erläutert. Die Prozentangaben beziehen sich auf das Gewicht, wenn nicht anders angegeben.

**Beispiel 1**

Haltbarkeitsuntersuchung von Salat-Mayonnaise

Es wurde eine Salat-Mayonnaise nach folgender Rezeptur hergestellt:

| | |
|---|---|
| Öl | 50,0 % |
| Wasser | 31,1 % |
| Stärke | 3,1 % |
| Eigelb | 4,6 % |
| Salz | 1,0 % |
| Zucker | 4,0 % |
| Guarmehl | 0,2 % |

Der pH-Wert der Salat-Mayonnaise wurde mit Essigsäure auf pH 4,5 eingestellt. Der besatz wurde in vier Chargen unterteilt, von denen eine unverändert belassen wurde (I). Den anderen Teilmengen wurden jeweils 100 U Lysozym pro mg (II), 50 U Lysozym pro mg + 0,15 % Kaliumsorbat (III) und 0,2 % Kaliumsorbat (IV) zugesetzt.

Die Lagerung der Ansätze erfolgte bei einer Temperatur von +10°C in verschlossenen Gläsern.

Die Haltbarkeit wurde anhand sensorisch wahrnehmbarer Veränderungen sowie der Gesamtkeimzahl beurteilt.

| Konservierungsstoff-Zusatz | Haltbarkeit [Tage] |
|---|---|
| I Kontrollversuch | 6 |
| II 100 U Lysozym/mg | 12 |
| III 50 U Lysozym/mg + 0,15 % Kaliumsorbat | 19 |
| IV 0,2 % Kaliumsorbat | 14 |

Aktivitätsbestimmung von bakterienlysierendem Enzymprodukt: Zu 2,8 ml einer Suspension von 0,2 mg Micrococcus luteus ATCC 4698 (Boehringer Mannheim) pro ml 0,1 M Natriumacetatpuffer (pH 5,0) werden 0,2 ml bakterienlysierendes Enzymprodukt-haltige Proben pipettiert und bei 25°C die Abnahme der Trübung durch Messung der Extinktion bei 450 nm bestimmt. Als 1 U wird die Extinktionsabnahme von 0,001 Photometer-Skalaeinheiten pro Minute definiert.

**Beispiel 2**

Haltbarkeitsuntersuchung von Mayonnaise

Es wurde eine Mayonnaise nach folgender Rezeptur hergestellt:

Öl      82,0 %
Wasser  10,0 %
Zucker  4,0 %
Senf    2,0 %
Salz    2,0 %

Der pH-Wert der Mayonnaise wurde mit Essigsäure auf 4,5 eingestellt. Die Mayonnaise wurde analog zu Beispiel 1 behandelt, gelagert und beurteilt. Es ergaben sich folgende Haltbarkeiten:

| | Konservierungsstoff-Zusatz | Haltbarkeit [Tage] |
|---|---|---|
| I | Kontrollversuch | 9 |
| II | 100 U Lysozym/mg | 15 |
| III | 50 U Lysozym/mg + 0,15 % Kaliumsorbat | 23 |
| IV | 0,2 % Kaliumsorbat | 17 |

**Beispiel 3**

Haltbarkeitsuntersuchung von Fleischsalat

Die in Beispiel 1 hergestellten Salat-Mayonnaise I - IV wurden mit jeweils 40 Gew.-% feingeschnittener Fleischwurst und 20 Gew.-% feingeschnittenen Salzgurken einer Herstellungscharge vermengt. Die Fleischsalate wurden analog zu Beispiel 1 behandelt, gelagert und beurteilt. Es ergaben sich folgende Haltbarkeiten:

| | Konservierungsstoff-Zusatz | Haltbarkeit [Tage] |
|---|---|---|
| I | Kontrollversuch | 3 |
| II | 40 U Lysozym/mg | 6 |
| III | 20 U Lysozym/mg + 0,06 % Kaliumsorbat | 11 |
| IV | 0,08 % Kaliumsorbat | 8 |

**Beispiel 4**

Haltbarkeitsuntersuchung von Salat-Dressing

Es wurde ein Salat-Dressing nach folgender Rezeptur hergestellt:

Wasser  34,1 %
Öl      46,1 %
Zucker  14,5 %
Senf    3,0 %
Salz    2,1 %
Xanthan 0,2 %

Der pH-Wert wurde mit Essigsäure auf 4,5 eingestellt. Das Salat-Dressing wurde analog zu Beispiel 1 behandelt, gelagert und beurteilt. Es ergaben sich folgende Haltbarkeiten:

|  | Konservierungsstoff-Zusatz | Haltbarkeit [Tage] |
|---|---|---|
| I | Kontrollversuch | 6 |
| II | 100 U Lysozym/mg | 10 |
| III | 50 U Lysozym/mg + 0,15 % Kaliumsorbat | 17 |
| IV | 0,2 % Kaliumsorbat | 12 |

**Beispiel 5**

Haltbarkeitsuntersuchung einer Creme-Füllung

Es wurde eine Kuchencreme nach folgender Rezeptur hergestellt:

| Zucker | 51,2 % |
|---|---|
| Glukosesirup | 25,6 % |
| Wasser | 22,0 % |
| funktionalisiertes Protein | 0,7 % |
| Citronensäure | 0,4 % |
| Agar-Agar | 0,07 % |
| Aromastoffe | 0,03 % |

Die Creme wurde in vier Chargen unterteilt, von denen eine unverändert belassen wurde (I). Den anderen Teilmengen wurden jeweils 100 U Lysozym pro mg (II), 50 U Lysozym pro mg + 0,15 % Kaliumsorbat (III) und 0,2 % Kaliumsorbat (IV) zugesetzt.

Die Lagerung der Ansätze erfolgte bei einer Temperatur von +10°C in verschlossenen Gläsern.

Die Haltbarkeit wurde anhand sensorisch wahrnehmbarer Veränderungen sowie der Gesamtkeimzahl beurteilt.

|  | Konservierungsstoff-Zusatz | Haltbarkeit [Tage] |
|---|---|---|
| I | Kontrollversuch | 5 |
| II | 100 U Lysozym/mg | 9 |
| III | 50 U Lysozym/mg + 0,15 % Kaliumsorbat | 15 |
| IV | 0,2 % Kaliumsorbat | 11 |

**Beispiel 6**

Haltbarkeitsuntersuchung einer geschäumten Fruchtdessert-Speise

Es wurde eine Fruchtdessert-Speise nach folgender Rezeptur hergestellt:

| Wasser | 74,6 % |
|---|---|
| Puderzucker | 18,8 % |
| modifizierte Stärke | 3,7 % |
| Instant-Gelatine | 1,2 % |
| Fruchtpulver | 0,7 % |
| Citronensäure | 0,4 % |
| Glucono Delta Lacton | 0,3 % |
| Natriumhydrogencarbonat | 0,1 % |
| funktionalisiertes Protein | 0,1 % |
| Aromastoffe | 0,1 % |

Die Fruchtdessert-Speise wurde in vier Chargen unterteilt, von denen eine unverändert belassen wurde (I). Den anderen Teilmengen wurden jeweils 100 U Lysozym pro mg (II), 50 U Lysozym pro mg + 0,15 % Kaliumsorbat (III) und 0,2 % Kaliumsorbat (IV) zugesetzt.

Die Lagerung der Ansätze erfolgte bei einer Temperatur von +10°C in verschlossenen Gläsern.

Die Haltbarkeit wurde anhand sensorisch wahrnehmbarer Veränderungen sowie der Gesamtkeimzahl beurteilt.

| Konservierungsstoff-Zusatz | Haltbarkeit [Tage] |
|---|---|
| I Kontrollversuch | 6 |
| II 100 U Lysozym/mg | 10 |
| III 50 U Lysozym/mg + 0,15 % Kaliumsorbat | 16 |
| IV 0,2 % Kaliumsorbat | 11 |

**Beispiel 7**

Haltbarkeitsuntersuchung einer Feuchtigkeitscreme

Es wurde eine Feuchtigkeitscreme nach folgender Rezeptur hergestellt:

| | |
|---|---|
| Paraffinöl DAB 7 | 10,0 % |
| Vitaminöl | 5,0 % |
| Vaseline DAB 7 | 5,0 % |
| ®Hostacerin T-3 (Fettalkoholpolyglykol-ether, Emulgator) | 5,0 % |
| ®Hostaphat KW 340 N (organisches Phosphor-säurederivat, Emulgator) | 4,0 % |
| Palmitinsäure | 5,0 % |
| Cetylalkohol DAB 7 | 1,0 % |
| Wasser | 60,0 % |
| ®Hydroviton (Feuchthaltemittel) | 5,0 % |

Die Creme wurde in vier Chargen unterteilt, von denen eine unverändert belassen wurde (I). Bei der Herstellung der Cremes II - IV wurden der wäßrigen Phase folgende Zusätze zudosiert:

Creme II       250 U Lysozym/mg
Creme III     150 U Lysozym/mg + 0,2 % Sorbinsäure
Creme IV     0,3 % Sorbinsäure

Die Lagerung der Cremes erfolgte bei Raumtemperatur in verschlossenen Gefäßen.

Die Haltbarkeit wurde anhand sensorisch wahrnehmbarer Veränderungen sowie Gesamtkeimzahl beurteilt.

| Konservierungsstoff-Zusatz | Haltbarkeit [Tage] |
|---|---|
| I Kontrollversuch | 9 |
| II 250 U Lysozym/mg | 14 |
| III 150 U Lysozym/mg + 0,2 % Sorbinsäure | 30 |
| IV 0,3 % Sorbinsäure | 19 |

6

**Beispiel 8**

Haltbarkeitsuntersuchung eines Gels

Es wurde ein pharmazeutisches Gel nach folgender Rezeptur hergestellt:

```
Wasser                                      97 %
      Hydroxyethylcellulose (Tylose H 1000)    3 %
```

Das hergestellte Gel wurde in vier Chargen unterteilt, von denen ein Gel (I) unbehandelt blieb. Den Gelen II - IV wurden folgende Zusätze zudosiert:
Gel II        250 U Lysozym pro mg
Gel III       150 U Lysozym pro mg + 0,2 % Sorbinsäure
Gel IV        0,3 % Sorbinsäure
Die Lagerung der Gele erfolgte bei Raumtemperatur in verschlossenen Gefäßen.
Die Haltbarkeit wurde anhand sensorisch wahrnehmbarer Veränderungen sowie der Gesamtkeimzahl beurteilt.

| Konservierungsstoff-Zusatz | Haltbarkeit [Tage] |
|---|---|
| I   Kontrollversuch | 7 |
| II   250 U Lysozym/mg | 19 |
| III  150 U Lysozym/mg + 0,2 % Sorbinsäure | 35 |
| IV   0,3 % Sorbinsäure | 22 |

**Beispiel 9**

Haltbarkeitsuntersuchung von Flüssiglab

Zur Haltbarkeitsuntersuchung wurde im Handel erhältliches Flüssiglab verwendet, das mit 1,1 % Benzoesäure konserviert war. Das Flüssiglab wurde ohne und mit Zusatz von 50 000 U Lysozym pro ml bei Raumtemperatur in geschlossenen Gefäßen gelagert.
Die Haltbarkeit wurde anhand sensorisch wahrnehmbarer Veränderungen sowie der Gesamtkeimzahl beurteilt.

| Konservierungsstoff-Zusatz | Haltbarkeit [Tage] |
|---|---|
| 1,1 % Benzoesäure | 16 |
| 50 000 U Lysozym/ml + 1,1 % Benzoesäure | 33 |

**Patentansprüche**

1. Zubereitung enthaltend Benzoesäure und/oder Sorbinsäure oder deren Salze sowie ein oder mehrere Enzyme mit N-Acetylmuramidaseaktivität in einem Gewichtsverhältnis von 1 zu 100 bis 100 zu 1.

2. Zubereitung nach Anspruch 1, dadurch gekennzeichnet, daß Benzoesäure und/oder Sorbinsäure oder deren Salze und das Enzym mit N-Acetylmuramidaseaktivität in einem Gewichtsverhältnis von 1 zu 50 bis 50 zu 1 gemischt werden.

3. Zubereitung nach Ansprüch 1 oder 2, enthaltend Benzoesäure und/oder Sorbinsäure oder deren Salze

7

sowie Lysozym und/oder bakterienlysierende Enzymprodukte aus Streptomyceten.

4. Verwendung der Zubereitung nach einem oder mehreren der Ansprüche 1 bis 3 als Konservierungsmittel.

5. Verwendung nach Anspruch 4 in Lebensmitteln, kosmetischen und pharmazeutischen Produkten, Verpackungsmaterialien, Tabak und Tabakerzeugnissen sowie Futtermittel.

6. Verwendung nach Anspruch 4 oder 5, dadurch gekennzeichnet, daß dem zu konservierenden Produkt die Zubereitung in einer Menge zugesetzt wird, daß 0,01 bis 2000 U des Enzyms mit N-Acetylmuramidaseaktivität pro mg darin enthalten sind.

7. Verwendung nach Anspruch 6, dadurch gekennzeichnet, daß 0,1 bis 1000 U/mg enthalten sind.

8. Verfahren zur Herstellung der Zubereitung nach einem oder mehreren der Ansprüche 1 bis 3, dadurch gekennzeichnet, daß Benzoesäure und/oder Sorbinsäure oder deren Salze zusammen mit dem Enzym mit N-Acetylmuramidaseaktivität in eine geeignete Applikationsform gebracht werden.

## Claims

1. A formulation containing benzoic acid and/or sorbic acid or their salts plus one or more enzymes having N-acetylmuramidase activity in a ratio by weight of 1 to 100 to 100 to 1.

2. A formulation as claimed in claim 1, wherein benzoic acid and/or sorbic acid or their salts and the enzyme having N-acetylmuramidase activity are mixed in a ratio by weight of 1 to 50 to 50 to 1.

3. A formulation as claimed in claim 1 or 2, containing benzoic acid and/or sorbic acid or their salts, plus lysozyme and/or bacteriolytic enzyme products from Streptomycetes.

4. The use of the formulation as claimed in one or more of claims 1 to 3 as preservative.

5. The use as claimed in claim 4 in food products, cosmetic and pharmaceutical products, packaging materials, tobacco and tobacco products, as well as animal feeds.

6. The use as claimed in claim 4 or 5, wherein the amount of the formulation added to the product to be preserved is such that it contains 0.01 to 2000 U of the enzyme having N-acetylmuramidase activity per mg.

7. The use as claimed in claim 6, wherein the content is 0.1 to 1000 U/mg.

8. A process for the preparation of the formulation as claimed in one or more of claims 1 to 3, which comprises converting benzoic acid and/or sorbic acid or their salts together with the enzyme having N-acetylmuramidase activity into a suitable application form.

## Revendications

1. Composition contenant de l'acide benzoique et/ou de l'acide sorbique ou des sels de ceux-ci et une ou plusieurs enzymes possèdant une activité de N-acétylmuramidase, en une proportion en poids allant de 1 pour 100 à 100 pour 1.

2. Composition selon la revendication 1, caractérisée en ce que l'acide benzoïque et/ou l'acide sorbique ou des sels de ceux-ci et l'enzyme possèdant une activité de N-acétylmuramidase sont mélangés en une proportion en poids allant de 1 pour 50 à 50 pour 1.

3. Composition selon la revendication 1 ou 2, contenant de l'acide benzoïque et/ou de l'acide sorbique ou des sels de ceux-ci et du lysozyme et/ou des produits enzymatiques bactériolytiques dérivés des streptomycètes.

4. Utilisation de la composition selon une ou plusieurs des revendications 1 à 3 comme agent de conservation.

5. Utilisation selon la revendication 4 dans les aliments, les produits cosmétiques et pharmaceutiques, les matières d'emballage, le tabac et les produits à base de tabac, et dans le fourrage.

6. Utilisation selon les revendications 4 ou 5, caractérisé en ce que l'on ajoute la composition au produit à conserver en une quantité contenant 0,01 à 2000 U d'enzymes possèdant une activité de N-acétylmuramidase par mg.

7. Utilisation selon la revendication 6, caractérisée en ce que sont présentes 0,1 à 1000 U/mg.

8. Procédé pour préparer la composition selon une ou plusieurs des revendications 1 à 3, caractérisé en ce que l'on met l'acide benzoïque et/ou l'acide sorbique ou des sels de ceux-ci, conjointement avec l'enzyme possèdant une activité de N-acétylmuramidase, sous une forme d'application appropriée.